Europäisches Patentamt

European Patent Office

Office européen des brevets

(11) Publication number: 0 302 148
A1

## (12) EUROPEAN PATENT APPLICATION

(21) Application number: 87306852.2

(22) Date of filing: 03.08.87

(51) Int. Cl.⁴: **A61N 1/36** , **A61F 5/01**

(43) Date of publication of application:
**08.02.89 Bulletin 89/06**

(84) Designated Contracting States:
**AT BE CH DE ES FR GB GR IT LI LU NL SE**

(71) Applicant: **University of Strathclyde**
**McCance Building 16 Richmond Street**
**Glasgow G1 1XW(GB)**

(72) Inventor: **Andrews, Brian**
**Wolfson Centre University of Strathclyde**
**Glasgow G4 0NW(GB)**

(74) Representative: **McCallum, William Potter et al**
**Cruikshank & Fairweather 19 Royal**
**Exchange Square**
**Glasgow G1 3AE Scotland(GB)**

(54) **Hybrid orthosis.**

(57) A functional electrical stimulation orthosis for restoring locomotion in neuroligically impaired sujects, such as, is described. The orthosis has knee locking means (20, 34) for securement to each leg for enforcing knee extension in a standing position, and a sensor (36) located on each leg for sensing knee flexation. A plurality of electrodes (42,44; 44,46 and 46,48) are disposed on each leg to stimulate extension and flexion movement under the control of a control means (38). The orthosis can be used with crutches and stimulation of flexion and extension movement can be effected manually or automatically using an open-loop or closed-loop system. Various embodiments and modifications of the invention are disclosed with other orthosis such as a foot ankle orthosis (12) to provide a hybride orthosis.

FIG.1

# HYBRID ORTHOSIS

The present invention relates to functional electric stimulation orthoses for restoring locomotion in neurologically impaired patients such as paraplegics.

Functional electrical stimulation (F.E.S.) orthoses are devices which include a mechanical structure which support paraplegic limbs, usually lower limbs, and a plurality of electrodes adapted to overlie muscles of the paraplegic limbs and which can be energised in a predetermined sequence to stimulate the muscles to cause certain movements of the limbs to provide a variety of functions, such as walking, standing or sitting. However, although there are many such devices most of them are impractical because they do not permit satisfactory simulation of the normal locomotion. Some existing orthoses permit a cumbersome waddling-type gait which is not only tiring for the paraplegic but the type of movement is unnatural and is difficult to control.

A functional electrical stimulation orthosis should satisfy a number of basic criteria in addition to being inexpensive, easy to manufacture and reliable. When used with paraplegics it should be capable of causing movement to resemble normal locomotion and to be used with or without walking aids depending on the degree of paraplegia. It should require minimal hardware and be relatively lightweight to minimise the impediments to paraplegic gait. In addition, the orthosis should be completely controllable by the user and should permit a number of different movement activities such as standing up, walking on a flat surface, walking up and down stairs, standing up straight and sitting down from a standing up position. In addition, in the event of failure of the control system the mechanical components should be capable of providing mechanical stability for the paraplegic when in the standing position and use of the F.E.S. orthosis should avoid premature muscle fatigue.

An object of the present invention is to obviate or mitigate disadvantages associated with the aforementioned functional electrical stimulation orthosis.

Accordingly, in one aspect of the invention there is provided a functional electrical stimulation orthosis for restoring locomotion in neurologically impaired patients comprising knee locking means adapted to be secured to each leg of a paraplegic patient for enforcing knee extension in a standing position;

sensor means located on each leg for sensing knee flexion of that respective leg;

control means coupled to each sensor means for receiving sensor signals produced therefrom in response to knee flexion, said control means having desired movement selector means actuable by the patient for providing output control signals for providing a desired movement by the patient;

first electrode means coupled to said sensor means and adapted to be associated with a knee extension reflex mechanism for providing electrical stimulii to said knee extension muscles in response to a first control signal from said control means;

second electrode means coupled to said sensor means and adapted to be associated with a knee flexion reflex mechanism for providing electrical stimulii to said knee flexion reflex mechanism in response to a second control signal from said control means;

the arrangement being such that, in use, when said desired movement selector means is actuated for a desired stance situation, knee extension and patient stability is maintained by said control means responding to knee flexion signals from each of said sensor means by providing said first control signal to said first electrodes to provide electrical stimulation of said knee extension reflex mechanism, and when said user selection control means are selected to provide desired locomotion, said knee extension control signal to one leg is disabled and said second control signal is provided to said second electrode means to electrically stimulate said knee flexion reflex of the same leg to permit the leg to swing forward and allow locomotion.

Preferably, the knee locking means consists of an ankle and calf support and a thigh support coupled together by hinge means, the thigh support being proportioned such that, in use, in the stance condition, the hinge means is disposed rearwardly of the natural knee joint so that the line of action due to body weight passes in front of the hinge means.

Preferably also, the sensor means is a microswitch associated with the hinge means, the microswitch being normally closed in full knee extension. Alternatively, the sensor means may be potentiometers or any suitable sensor responsive to angular displacement such as a goniometer.

In an alternative embodiment, the knee locking means is a resilient ankle and calf support with the line of action of the bodyweight vector, that is, the vertical line through the centre of gravity of the body causing a resilient reaction force in the ankle and calf support to provide a knee locking force. Preferably also, the sensor means is a heel switch disposed in the base of the ankle and calf support and is responsive to heel lift as indicative of knee flexion to provide a sensor signal to said control means.

Alternatively, the sensor means in the alternative arrangement is a strain gauge for sensing strain in said ankle and calf support. Also, in the alternative embodiment a pressure sensor may be located under the ball of the foot to avoid potential instability just to the heel raised from the floor to permit the knee extensors and/or hip extensors which extend the knee joint, to be stimulated a predetermined time in advance of when they are normally stimulated.

Preferably, four electrodes are used to provide electrical stimulation of each leg and these are arranged in three pairs each requiring three control channels, the electrodes being located at,

(a) the side of the gluteal muscles (medius and maximus) to give hip extension and pelvic stabilisation;

(b) upper anterior thigh for quadraceps stimulation;

(c) lower anterior thigh for quadraceps stimulation;

(d) over the anterior head of the fibula at the side of the peroneal nerve,    electrodes (a) and (b) are used for gluteal stimulation, electrodes (b) and (c) are used for quadraceps stimulation and electrodes (c) and (d) are used for stimulation of the common peroneal nerve flexion withdrawal reflex. Preferably the electrodes are surface electrodes. Alternatively the electrodes are implantable electrodes.

Alternatively, the knee locking means may be provided by a brake or clutch mechanism, for example a solenoid and spring loaded bail lock or a magnetic particle brake or a mechanical clutch with Bowden cable from crutch hand grips. Preferably, the orthosis could be used with crutches and stimulation control means incorporated into the crutch hand grips.

Preferably said knee locking means includes position sensing means for determining the position of a floor reaction vector (FRV), and said position sensing means providing a signal to said first electrode means when said FRV passes within a certain distance of the knee joint axis. Preferably also said position sensing means consists of a patella pad coupled to the ankle and calf support by a strap, the tension in said pad being used to determine the position of thr FRV. Conveniently said tension is sensed by displacement of a resilient spring loaded switch disposed in line with the pad and coupled to the ankle and calf support.

Preferably said resilient spring loaded switch has a compression spring and said spring tension is adjustable to set a threshold tension, so that an electrical signal is sent to said first electrode means, when a predetermined threshold is reached.

Conveniently the spring loaded switch is in series with the stimulation output to the quadraceps whereby stimulus to the quadraceps is automatically delivered to the quadraceps whenever the strap tension falls below the threshold.

In another aspect of the invention there is provided a floor reaction hybrid orthosis comprising, a floor-reaction foot-ankle orthosis, and an FES control system, said FES control system including first electrode means adapted to be disposed on said quadraceps for stimulation thereof to provide knee extension, and second electrode means adapted to be disposed in proximity to an afferent nerve for activating said knee tension, floor reaction vector (FRV) position sensing means for sensing the position of said floor reaction vector and stimulating said quadraceps via said first electrode means when said FRV passes within a predetermined distance of said knee joint axis.

This is achieved by using a patella pad and resilient spring loaded strap coupled between the pad and the orthosis. The arrangement being such that when the strap tension exceeds a preset threshold, indicative that the FRV is approaching the knee axis, the switch trips and a signal is sent to said first electrode means to stimulate the quadraceps.

Embodiments of the present invention will now be described by way of example, with reference to the accompanying drawings in which:-

Fig. 1 is a diagrammatic side elevation of a right leg carrying a first embodiment of an F.E.S.orthosis according to the invention;

Fig. 2 is a view similar to Fig. 1 of a second embodiment according to the invention;

Figs. 3a, b and c are diagrammatic side elevations of the orthosis shown in Fig. 1 with a different knee locking mechanism;

Fig. 4 depicts a hybrid orthosis for use in assisting prolonged standing and walking in neurologically impaired patients, and Fig. 5 depicts an alternative embodiment of a hybrid orthosis similar to that shown in Fig. 4 but with an additional thigh support and set of knee hinges.

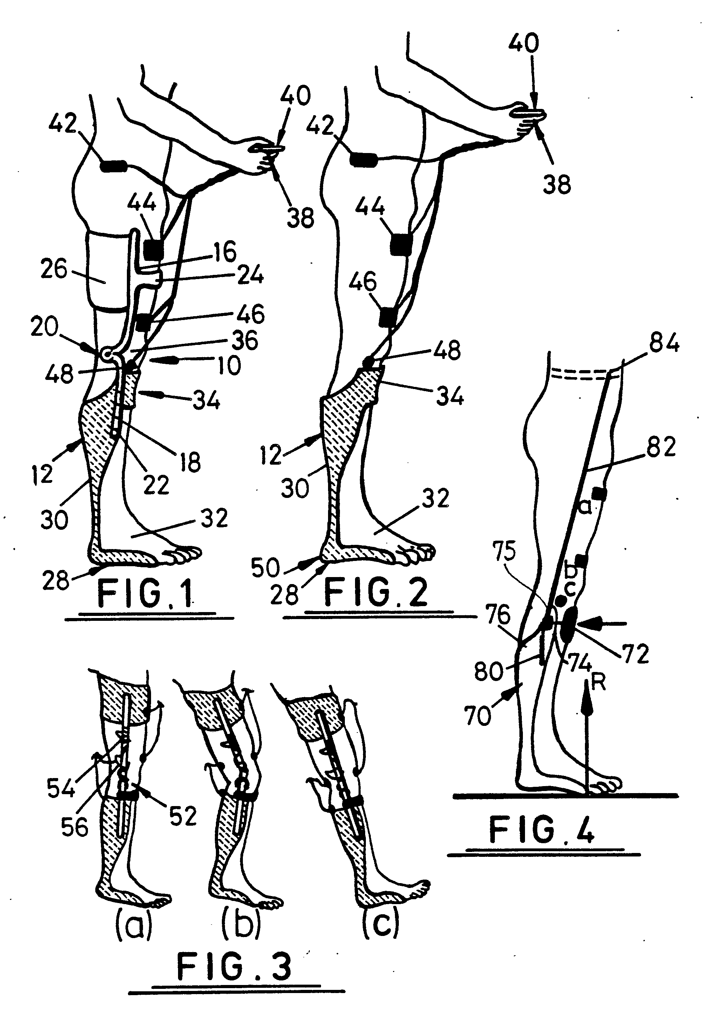

Reference is first made to Fig. 1 of the drawings which illustrates a first embodiment of functional electrical stimulation orthosis. It will be understood that although only one leg is shown in parts to be described and which are illustrated are also applied to the left leg. The F.E.S. comprising a leg support brace generally indicated by reference numeral 10 which consists of a lower moulded plastic resilient calf and ankle support 12 and an upper regid metallic thigh support generally indicated by reference numeral 14. The upper thigh support consists of a first upwardly extending elongate portion 16 and a lower downwardly extending elongate portion 18 which is joined to the portion 16 by a hinge 20. The lower portion 18 is fastened to the orthosis 12 by means of screws (not shown) which are received by drilled holes 22 in the portion 18. The upper portion 16 has an integral anteriorly extending thigh support bar 24 which joins with a like upper thigh support on the inside of the leg (not shown). A posterior thigh support 26 made from fabric is also attached to upper portion 16 for relieving pressure during movement and is one of the areas designed to give three point support. The fabric support deforms during sitting to avoid potential pressure sores or tissue damage problems.

The ankle and calf support 12 consists of a foot support portion 28 which is integral with a calf support portion 30 and which prevents the calf support portion from slipping up and down the leg during locomotion. The support is flexible around the ankle joint 32 and does not significantly impede movement by the paraplegic. The orthosis has an integral knee band generally indicated by reference numeral 34 which extends across the anterior portion of the leg just below the knee at the level at the top of the fibula and tibia. Because support 12 is resilient the weight of the paraplegic pushes against the knee band causing a reaction force due to resilience of the knee band which tends to lock the knee and stabilise the paraplegic when standing.

It will be seen that the joints 20 are offset rearwardly from the upper and lower elongate portions and behind the natural knee joint axis. The line of action of body weight passes in front of the hinge joints 20 for standing so that throughout most of the stance phase this joint will maintain the natural knee joint and stable knee extension. The hinge joint also incorporates a microswitch sensor or any other suitable sensor such as a rotary potentiometer, to detect when the joint 20 is fully extended or flexed and this sensor is used by the control system as will be later described to control the application of stimulii to the muscles of the paraplegic to provide muscle stimulation for a desired movement.

A hand held control unit 38 has a plurality of selector switches 40 located thereupon for actuation by the user to form a desired movement. It will be appreciated that although this is shown as a hand held unit it could readily by incorporated into the arm of a crutch.

The control unit is connected via conductors to a plurality of electrodes located at the strategic positions over the paraplegic's limb to permit stimulation of extensor and flexor reflex mechanisms to cause a variety of movements to occur. Four electrodes are shown in this embodiment and these are sufficient to permit a variety of different movements to be obtained. A gluteal electrode 42 is located over the gluteal medius and maximus muscles so that when stimulated gives hip extension and pelvic stabilisation. An upper thigh electrode 44 is located over the anterior portion of the thigh and when energised stimulates the upper quadraceps and also doubles for gluteal stimulus with electrode 42, as will be explained. A lower quadraceps electrode 46 stimulates the lower quadraceps when energised and also functions with peroneal electrode 48 for stimulation of the peroneal nerve to actuate the flexion withdrawal mechanism as will also be explained.

Electrode pairs, 42 and 44, 44 and 46, and 46 and 48, may be stimulated through a respective single channel thus requiring three channel stimulation controlled by the control unit. This will be best explained by reference to the different locomotion functions and movement requirements to be carried out by the paraplegic.

It will be understood that the individual channels of the stimulator are known per se and that the nature and magnitude of the signals required to stimulate the individual muscles and nerves mentioned are also known as are the type of electrodes and electrode material.

Operation of the apparatus of Fig. 1 may be best explained by reference to different desired movement situations which illicit different control signals and logic from the control unit 38.

Firstly, movement of the paraplegic from a sitting to a standing position, this will now be described. In the sitting position no stimulation is applied and the knees are flexed. When the paraplegic wishes to stand he first moves to the edge of the seat and positions his feet appropriately on the floor. He then presses a control switch marked "STAND" and, after a delay of approximately four seconds to permit the paraplegic to adjust his forward leaning posture and to hold on to supports if necessary, for example if he is sitting in a wheelchair, stimulii is then applied to the knee extensor muscles, i.e. the quadraceps, via electrodes 44 and 46. Because of the configuration of his body contraction of the quadraceps cause him to be raised to the standing position as shown in Fig. 1. The paraplegic can assist in this movement by taking part of his body weight on his arms if there are arm rests available.

In the standing position as shown in Fig. 1, the knee joints are in full extension as sensed by the microswitches in the joints 20 and the stimulus is removed shortly afterwards. Stability is maintained in the upright standing position due to two factors. The first factor is by the mechanical force exerted by the resilient knee band of the calf and ankle support 12 and the second factor is the nature of the offset hinge 20 which lies behind the line of action of the body weight so that inadvertent flexion of the knee does not result in collapse of the paraplegic. Therefore, during standing no stimulus is required. However, should the knee inadvertently flex so that the microswitch 36 is opened this is sensed by the control unit 38 which in turn sends a signal to electrodes 44 and 46 to electrically stimulate and extend the quadraceps and cause the knee to extend until the microswitch 36 closes and registers the knee joints as being fully extended. In this way the reflex loop is maintained and stability is automatically maintained.

Sitting is the reverse process and in this case when the paraplegic desires to sit he presses the "SIT" switch and electrical stimulii is then applied to the quadraceps for slowly decreasing amplitude via electrodes 44 and 46. The paraplegic responds to this stimulus by leaning backwards and flexing his knees and slowly returns to his seat. The slowly decreasing stimulii to the quadraceps causes some contraction of the quadraceps sufficient to compensate for the body weight during sitting so that the paraplegic does not simply collapse in the seat but rather slowly returns to the seat. The automatic action of the quadraceps coming on in response to the knee opening may be thought of as an artificial reflex which stimulates the normal reflex mechanism.

During walking the paraplegic may use crutches and, in this case, the control system uses two additional hand controls, one mounted on each crutch handle. Crutches are not shown in the interests of clarity. Also, two additional channels of stimulii are used per leg. In order for the subject to make a step forward the paraplegic firstly transfers his body weight to the stance limb and the stance limb stability is maintained by the offset knee joint and, should the need arise, by the automatic knee extension reflex. The subject then presses a switch on the control unit which inhibits the automatic knee extension reflex of the other leg and stimulii is simultaneously applied to the peroneal nerve via the peroneal nerve electrode 48 on the swing leg which illicits the flexion withdrawal reflex causing simultaneous reflexion at the hip, knee and ankle and to the gluteal muscles of the stance limb to stabilise the pelvis. However, it should be noted that pelvic stability can be obtained in some paraplegics without the requirement of gluteal stimulus by the paraplegic using the latissimus dorsi and trapezius muscles and applying stabilising force actions through the crutches. The use of gluteal stimulus, however, reduces the effort required of the upper limb. The gluteal muscles of the swing limb may also be stimulated using electrodes 42 and 44 to provide abducting action which is sometimes necessary in paraplegics with adductor spasticity to ensure the correct positioning of the foot at the end of the swing phase. This may also be dealt with by using a mechanical linkage between the legs that prevents them from coming too close together whilst not restricting the other required motions.

During walking, when the paraplegic removes his finger from the knee extension inhibit control switch the knee extension reflex enables the control unit 38 to send stimulii to the knee extensors through electrodes 44 and 46 causing the knee to extend just before heel strike. The gluteal stimulus is maintained until shortly after heel strike whereupon the paraplegic transfers body weight to the stance limb and the cycle repeats. The left and right leg alternate the role of stance limb to give a reciprocal gait pattern.

It will be appreciated that stair or step climbing activity using the orthosis with crutches may be possible and in this case body weight is tranferred by the paraplegic to his stance limb and the other leg is flexed to land the foot on the next step and then apply knee extension and shifting the body weight to the stance leg for flexing the other leg to lift it on to the same step or the next step. The orthosis can have a leg brace on one side of the leg only.

Reference is now made to Fig. 2 of the drawings which is substantially the same as Fig. 1 except that the upper thigh support 14 is not used. In this case only the lower ankle support 12 is used and the same electrodes 42 and 44, 46 and 48 are also used in identical positions to that shown in Fig. 1. However, the ankle and calf support 12 incorporate a heel switch generally indicated by reference numeral 50 in the base of the support. The presence of the heel switch enables the knee extension reflex to be implemented because if the heel is raised from the ground the switch opens and sends a signal to the control unit 38 which causes electrical signals to be sent to electrodes 44 and 46 to stimulate the quadraceps to extend the knee so that the heel returns to the ground. It will be appreciated that the ankle and calf support 12 is sufficiently rigid and resilient so that when the subject is upright and the knee flexes, the heel must rise with the foot pivoting about the toes and that the resilience is such that the band 34 assists in "locking" the knee in the extended position.

As with the first embodiment described with reference to Fig. 1, the electrode pairs for gluteal, quadraceps and peroneal stimulation are the same and similarly the paraplegic can also achieve pelvic stabilisation using the latissimus dorsi and trapezius muscles together with force actions transmitted through crutches. It will be appreciated that an advantage of the function of electrical stimulation is that paraplegic effort is considerably reduced. It will also be appreciated that the stimulus to the quadraceps stops shortly after the heel switch registers that the stability has been restored to avoid undue muscle fatigue.

Reference is now made to Fig. 4 of the drawings which depicts a hybrid orthosis consisting of an AFO 70, knee extensor electrodes (a) and (b), knee flexion electrode (c) and a patella pad 72 coupled via a strap 74 to a resiliently biased switch (75) mounted on the upright portion 76 of the AFO 70.

Electrode (b) was common to both channels acting as an indifferent electrode. Monophasic, rectangular pulses were used having a duration of 0.3ms a pulse repetition frequency of 20Hz and an amplitude adjustable up to 120 volts (measured with a 1kohm load). Stimulation of the knee extensors electrodes was delivered through two 41mm × 88mm self adhesive electrodes (Myocare type 6282m 3M Ltd.), labelled (a) and (b) in figure 4. When stimulating the common peroneal nerve a smaller active electrode was positioned just behind the head of fibula as shown in figure 4.

A semi-automatic, standing posture, stabilising control loop has been incorporated to avoid fatiguing the quadriceps. During reciprocal walking the FES system delivered appropriately patterned stimuli to the quadriceps muscles and the common peroneal nerve. The latter was used to elicit a flexion withdrawal reflex to initiate the swing phase of gait.

Standing up from a seated position was assisted by open-loop stimulation of the quadriceps in a manner similar to that described above. Once upright, the subject leans forward slightly so that the foot/floor reaction vector (FRV) passes the knee joint axis anteriorly as shown. In this case a moment is generated at the ankle joint by the floor reaction force (R). This moment is opposed by a force (F) due to the pressure applied by the patella pad 72. In this posture the AFO 70 stabilises the leg without the need for continued stimulation of quadriceps. However, should the direction of the FRV pass behind the knee joint axis the leg will destabilise. The position of the FRV relative to the knee joint axis can be sensed by measuring the tension generated in the patella pad restraining strap 74. The strap tension was used to control the application of quadriceps stimulation. When the strap tension falls below a preset level, indicating that the direction of the FRV is too close to the knee axis, the quadriceps are maximally stimulated until the strap tension again exceeds the preset level. This requires the subject to be aware of the stimulation and to make the required postural shift by leaning forwards. Subjects who have used this orthosis have had preserved sufficient sensation to be aware of the applied stimulus. Feedback may alternatively have been provided by stimulating a sensitive area of skin or by using some audio visual cue. In the HO the strap tension was sensed by the small displacements of a compression spring (not shown) mounted in line with the strap and affixed to the plastic AFO upright 76. The threshold tension was set by adjusting the displacement required to turn off the electrical switch(s). This switch was in series circuit with the stimulator output to quadriceps. In this way stimulus was automatically delivered to the quadriceps whenever the strap tension falls below threshold .

It has been previously determined that a minimum of four channels of electrical stimulus are required to synthesise a simple reciprocal gait pattern in paraplegics, KRALJ A., BAJD T., TURK R., KRAJNIK J. AND BENKO H. (1983). Gait restoration in paraplegic patients. A feasibility demonstration using multichannel surface electrode FES. J. Rehabil. R&D, 20, No. 1 (BPR10-38), p 3-20. During the stance phase, the knee extensor muscles were activated and the swing phase was accomplished by eliciting the flexion withdrawal reflex by stimulating an afferent nerve. A similar approach was used for the HO described here.

From the patient's control point of view, the gait cycle was divided into stance and swing phases. For each leg the transition from one phase into another was achieved by pressing a corresponding hand switch. These hand switches were mounted onto the handgrips of the forearm crutches or walking frame/rollator. When a switch was not pressed the stance phase control loop described above was enabled. In order to take a step forward, the subject first transfers his body weight onto the contralaterial supporting leg and presses the ipsilateral hand switch. Whilst the switch was pressed ipsilateral stimulation of quadriceps was disabled and the common peroneal nerve stimulated. Stimulation of this mixed nerve elicits a flexion withdrawal response producing dorsiflexion and eversion of the foot and flexion of the hip and knee. The amount of flexion was dependent upon the preset stimulus intensity. To terminate the swing phase prior to foot contact, the hand switch was released and the automatic control of quadriceps re-enabled. This resulted in the immediate stimulation of quadriceps, causing the knee to extend at foot contact. The quadriceps stimulus was maintained until mid stance when the patella strap tension again exceeded the threshold. The duration of the swing phase was regulated by the time of pressing the switch. Flexion may, optionally, by triggered using a conductive rubber insole switch,with the active element positioned in the region of the metatarsal joints. When the subject transfers his body weight onto the stance limb this action unloads the switch causing the quadriceps stimulus to be inhibited and the peroneal stimulus to be applied for a preset time interval. This preset interval was adjusted to suit the subjects preferred cadence. In order to prevent false triggering of flexion it was necessary to build in the condition that the metatarsal switch be unloaded for an uninterrupted period of 0.1s before allowing flexion.

Two patients with spinal cord lesions have so far been included in the trials of the HO. In both cases quadriceps restrengthening exercises were undertaken using cyclical stimulation in a manner similar described to that described above.

The first subject (K.D, male age 34yrs, mass 88kg, height 1.88m, lesion T5/6 complete, 4yrs post injury) used the HO bilaterally using the handgrip mounted switches to control flexion when walking with a rollator type walking aid. The second subject (G.D, male aged 22 yrs, mass 70kg, height 1.8m, incompletely lesioned at the level C6, 3yrs post injury) had one leg that was completely paralysed whilst the other had sufficient voluntary control to enable him to remain standing for a short time using forearm crutches without stimulation. He had previously used a 2 channel FES device described in BAJD T., ANDREWS B.J., KRALJ A., KATAKIS J. (1985). Restoration of walking in patients with incomplete spinal cord injuries by use of surface electrical stimulation. Prosthetics and Orthotics International, vol 9, No. 2, pp 109-111. This subject used the HO unilaterally for standing and walking in forearm crutches fitted with handgrip switches for flexion control. This patient preferred the option of automatic triggering of flexion for level ground walking. He used manual control, as he did with his 2 channel FES device when negotiating uneven ground and steps.

The preliminary trials indicate that the HO was effective in stabilising the knee joint when applied unilaterally or bilaterally. Stimulation of the quadriceps was significantly reduced prolonging standing times almost indefinitely. The wearing of an AFO is cosmetically acceptable and does not interfere with level ground walking. The FES assisted walking may require less energy than walking in knee ankle foot braces with locked knee because no hip hiking is necessary with active flexion. Finally, FES-assisted walking is more aesthetic than locking knee gait for the observer and is preffered by the patients. There may be a number of therapeutic benefits to be gained from the use of FES based orthois. These may include prevention of pressure sores, contractures, muscle atrophy and bone demineralisation.

It will be appreciated that various modifications may be made to the embodiments hereinbefore described without departing from the scope of the invention.

For example, with regard to the embodiment described with reference to Fig. 1, it will be understood that a different upper thigh support could be used to provide the knee locking mechanism and knee stability when in a stance position. This could be provided by the arrangements as shown in Figs. 3a, 3b and 3c in which a solenoid actuated mechanism 52 is provided for knee locking. It will be seen that in Fig. 3a which is the upright stance position, the knee mechanism 52 is locked and in order to walk a signal is sent to a solenoid 54 which retracts a lever 56 which rotates a link out of a locking position permitting the knee to flex as shown in Fig. 3b. At toe off and during the swing phase to the position as shown in Fig. 3c, which is heel strike, the knee reflex mechanism is initiated because of the signal from the knee mechanism indicating flexion causing the quadriceps to extend the knee at heel strike. It will be appreciated that other actuators may be used instead of the solenoid such a magnetic particle brakes or a mechanical clutch with Bowden cable from the crutch handgrips. It will also be understood that stimulus can be applied through surface, percataneous or implanted electrodes.

In addition, with regard to the embodiment shown in Fig. 2, it will be understood that means for detecting flexation could be provided other than by the heel switch 50, for example, a strain gauge could be located in the region of the ancle to detect strain in the ankle and calf support 12 and this signal used to initiate the knee extensor reflex mechanism to maintain stance stability. Also, a pressure sensor could be located under the ball of the foot at the toes to protect potential instability just prior to the heel being raised from the floor and this could be used to provide a signal to the control unit which would permit the quadraceps to stimulated about 200 milliseconds earlier than normal to improve stance stability.

In the hybrid orthosis (HO) shown in Fig. 4, the strap and/or the compression spring may be replaced by an elastomeric variable resistance transducer for example a Flexigauge (trade mark), Flexigage Ltd, Glasgow, Scotland which can be used to sense strap tension, and a comparator can be used to deliver automatically a stimulus whenever the strap tension falls below the threshold.

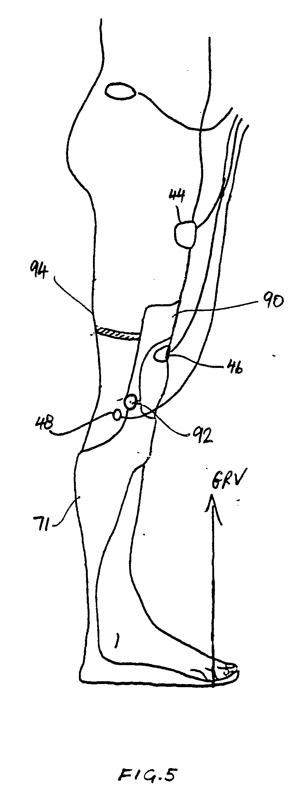

Reference is now made to Fig. 5 of the drawings which depicts an arrangement which minimises the possibility of causing joint laxity (genu recurvation)occuring in growing children due to continuous stretching of the cruciate knee ligaments because the knee extension in other normal situation tends to be limited by the tension in the posterior knee capsule and cruiate ligaments.

In the embodiment, which is somewhat similar to Fig. 2, the orthosis 1 is coupled to a moulded plastic upper thigh support 90 via a set of joints (two) 92 on the axis of the knee joint. The joints 92 allow full flexion of the knee but limit knee hyper-extension. The knee flexion senser can .readily be incorporated into the joint 92 and can be a microswitch or a potentiometer-goniometer as previously described.

A Velco (trade mark) strap 94 is used to retain the thigh support in contact with the skin surface during knee flexion ,for example, whilst sitting. It should also be understood that the Hybrid orthosis 71 is laminated in one piece and does not have a patella strap as shown in the Fig. 4 embodiment.

In this arrangement the knee extending movement generated by the ground reaction vecter (GRV) passing in front of the tree is balanced by the strains in the mechanical orthosis thigh support 90 and not by the posterior knee capsule or the cruciate ligaments. This also means that these strains, when measured, give direct information about the GRV relative to the knee joint. Therefore this information, together with the output of the knee Hexion senser, provides move reliable control of the quadraceps.

It will also be understood that the materials and components used herein are examplarly only and may be re placed by other like materials and components to provide a similar function. For example, the thigh support may be plastic.

It will be appreciated that the mounted crutch handle controls may be simple switches or devices giving a degree of proportional control, for example, a pressure transducer such that the harder it is pressed the greater the intensity of stimuli to the peroneal nerve and the greater limb reflex. This is useful in adjusting the stepping height to accommodate changes in terrain or obstacles and also it will be appreciated that the joint microswitches could equally be potentionmeters in which case proportional closed-loop control could be implemented.

In the embodiments hereinbefore described the system is open-loop insofar there is no feedback from the flexion reflex to terminate the flexion reflex and stimulate the extension reflex. Termination is carried out by the patient using the hand switch. In a closed loop system flexion is initiated by unloading the leg as sensed by a pressure switch under the metatarsals of each foot. Stimulus is then transmitted to the flexion electrodes as described above to cause hip and knee flexion. The stimulation is terminated when a particular hip angle is reached as sensed by using hip goniometers thus the system is closed-loop and operates automatically. The closed-loop system compensates for differences between patients and optimises stimulation and gait for each particular patient and avoids response fatigue. The hip goniometer may conveniently be in the form of a displacement potentiometer 80 mounted on the shank and inextensible flexible cord 82 connecting the displacement potentiometer to the patients waist (eg belt) 84 as seen in Fig. 4. This is also applicable to the embodiment shown in Figs. 1 to 3.

Also the hip abductors and flexors on the contralateral side may be stimulated during single support to stabilise the pelvis. A safety feature is that if the hip angle is not reached in a predetermined time the stimulus is removed and this is readily achieved using a comparator circuit.

Advantages of the embodiments of the apparatus are that they are inexpensive, simple to construct and install and offer minimal impediment to paraplegics during a variety of locomotion activities. In addition, the muscles are used only for short duration and antigravity support during standing is provided by the mechanics thus reducing the time before the onset of muscle fatigue. The mechanical arrangement in Fig. 1 provides a fail safe mechanism in the event of electrical disconnection or muscle fatigue because the offset hinges are behind the line of action of body weight thus providing inherent stability in the standing position. A further advantage is that the orthosis can be used for the paraplegic to move between an upright and sitting position without the need for other pieces of apparatus although it will be appreciated that use of such apparatus, such as crutches, may be of assistance.

The orthosis can also be used to train the posture of paraplegics. For example, a visual indication of stimulation to occur is given to the paraplegic who can then orient this body to facilitate movement by the F.E.S. when stimulation does occur. The embodiments of the orthosis hereinbefore described are intended for use with all neurologically impaired patients including paraplegics.

## Claims

1. A functional electrical stimulation orthosis for restoring locomotion in neurologically impaired patients comprising:

knee locking means (12, 20, 24) adapted to be secured to each leg of a paraplegic patient for enforcing knee extension in a standing position;

sensor means (36) located on each leg for sensing knee flexion of that respective leg;

control movement (38) coupled to each sensor means for receiving sensor signals produced therefrom in response to knee flexation, said control means (38) having desired movement selector means (40) actuable by the patient for providing output control signals for providing a desired movement by the patient;

first electrode means (44,46) coupled to said sensor means and adapted to be associated with a knee extension reflex mechanism for providing electrical stimulli to said knee extension muscles in response to a first control signal from said control means (88);

second electrode means (48) coupled to said sensor means (36) and adapted to be associated with a knee flexion reflex mechanism for providing electrical stimulii to said knee flexion reflex mechanism in response to a second control signal from said control means (38);

the arrangement being such that, in use, when said desired movement selector means (46) is actuated for a desired stance situation, knee extension and patient stability is maintained by said control means (38) responding to knee flexion signals from each of said sensor means (36) by providing said first control signal to said first electrodes (44,46) to provide electrical stimulation of said knee extension reflex mechanism, and when said user selection control means (40) are selected to provide desired locomotion, said knee extension control signal to one leg is disabled and said second control signal is provided to said second electrode means (48) to electrically stimulate said knee flexion reflex of the same leg to permit the leg to swing forward and allow locomotion.

2. A functional electrical stimulation orthosis as claimed in claim 1 wherein the knee locking means consists of an ankle and calf support (12) and a thigh support (24) coupled together by hinge means (20), the thigh support being proportioned such that, in use, the stance condition, the hinge means (20) is disposed rearwardly of the natural knee joint so that the line of action due to body weight passes in front of the hinge means (20).

3. A functional electrical stimulation orthosis as claimed in claim 1 or 2 wherein the sensor means (36) is a microswitch associated with the hinge means (20), the microswitch being normally closed in full knee extension.

4. A functional electrical stimulation orthosis as claimed in claim 1 or 2 wherein the sensor means (36) is a potentionmeters or any suitable sensor responsive to angular displacement such a goniometer.

5. A functional electrical stimulation orthosis as claimed in claim 1 wherein the knee locking means (12, 20, 24,) is a resilient ankle and calf support (12) with the line of action of the bodyweight vector, that is, the vertical line through the centre of gravity of the body causing a resilient reaction force in the ankle and calf support to provide a knee locking force.

6. A functional electrical stimulation orthosis as claimed in claim 5 wherein the sensor means (36) is a heel switch (50) disposed in the base of the ankle and calf support (12) and is responsive to heel lift as indicative of knee flexion to provide a sensor signal to said control means (34).

7. A functional electrical stimulation orthosis as claimed in claim 5 wherein the sensor means (36) in the alternative arrangement is a strain gauge for sensing strain in said ankle and calf support.

8. A functional electrical stimulation orthosis as claimed in any one of claims 5 to 7 wherein a pressure sensor may be located under the ball of the foot to avoid potential instability just prior to the heel raised from the floor to permit the knee extensors and/or hip extensors which extend the knee joint, to be stimulated a predetermined time in advance of when they are normally stimulated.

9. A functional electrical stimulation orthosis as claimed in any preceding claim wherein four electrodes (42,44,46,48) are used to provide electrical stimulation of each leg and these are arranged in three pairs (42,44; 44,46; and 46,48) each requiring three control channels, the electrodes being located at,

a) the side of the gluteal muscles (medius and maximus) to give hip extension and pelvic stabilisation;

b) upper anterior thigh for quadraceps stimulation;

c) lower anterior thigh for quadraceps stimulation;

d) over the anterior head of the fibula at the side of the peroneal nerve,

electrodes (a) and (b) are used for gluteal stimulation, electrodes (b) and (c) are used for quadraceps stimulation and electrodes (c) and (d) are used for stimulation of the common peroneal nerve flexation withdrawal reflex.

10. A functional electrical stimulation orthosis as claimed in claim 9 wherein the electrodes are surface electrodes.

11. A functional electrical stimulation orthosis as claimed in claim 9 wherein the electrodes are implantable electrodes.

12. A functional electrical stimulation orthosis as claimed in claim 5 wherein the knee locking means (12, 20, 24) is provided by a brade or clutch mechanism, for example a solenoid and spring loaded bail lock or a magnetic particle brade or a mechanical clutch with bowden cable from crutch hand grips.

13. A functional electrical stimulation orthosis as claimed in any preceding claim wherein the orthosis is used with crutches and stimulation control means incorporated into the crutch hand grips.

14. A functional electrical stimulation orthosis as claimed in any preceding claim wherein said knee locking means includes position sensing means for determining the position of a floor reaction vector (FRV), and said position sensing means providing a signal to said first electrode means when said FRV passes within a certain distance of the knee joint axis.

15. A functional electrical stimulation orthosis as claimed in claim 14 wherein said position sensing means consists of a patella pad (72) coupled to the ankle and calf support (70), by a strap (74), the tension in said strap (74) being used to determine the position of the FRV.

16. A functional electrical stimulation orthosis as claimedin claim 15 wherein said tension is senses by a displacement of a resilient spring loaded switch (75) disposed in line with the pad (74) and coupled to the ankle and calf support (70).

17. A functional electrical stimulation orthosis as claimed in claim 16 wherein said resilient spring loaded switch (75) has a compression spring and said spring tension is adjustable to set a theshold tension, so that an electrical signal is sent to said first electrode means (44,46) when a predetermined threshold is reached.

18. A functional electrical stimulation orthosis as claimed in claim 16 or claim 17 wherein the spring loaded switch (75) is in series with the stimulation output to the quadraceps whereby stimulus to the quadraceps is automatically delievered to the quadraceps whenever the strip tension falls below the threshold.

19. A floor reaction hybrid orthosis comprising, a floor reaction foot-ankle orthosis (70), and an FES control system, said FED control system including first electrode means (a,b,c) adapted to be disposed on said quadraceps for stimulation thereof to provide knee extension, and second electrode means (c) adapted to be disposed in proximity to an afferent nerve for activating said knee Hexion floor reaction vector (FRV) position sensing means (72,74) for sensing the position of said floor reaction vector and stimulating said quadraceps via said first electrode means (a,b,c) when said FRV passes within a predetermined distance of said knee joint axis.

20. A hybrid orthosis as claimed in claim 19 including a patella pad (72) and resilient spring loaded strap (74) coupled between the pad and the orthosis (70), the arrangement being such that when the strap tension exceeds a preset threshold, indicative that the FRV is approaching the knee axis, the switch trips (75) and a signal is sent to said first electrode means (a,b) to stimulate the quadraceps.

21. A functional electrical stimulation orthosis as claimed in claim 5 including thigh support (90) for limiting the knee extension movement coupled to a resilient ankle and calf support (71) by hinged joints (92) substantially on the axis of the knee joint for allowing flexion and preventing knee hyperextension.

22. A functional electrical stimulation orthosis as claimed in claim 21 wherein a knee flexion sensor is incorporated into said hinged joints (92).

23. A functional electrical stimulator orthosis as claimed in claim 21 or 22 wherein said thigh support means (90) is a moulded plastic support which is coupled to the thigh by a means (94) to maintain said thigh support means (90) in contact with the thigh during knee flexion.

FIG.1

FIG.2

FIG.3

FIG.4

FIG.5

| | European Patent Office | EUROPEAN SEARCH REPORT | Application Number |
|---|---|---|---|

EP 87 30 6852

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | NATIONAL AEROSPACE AND ELECTRONICS CONFERENCE, Dayton, 19th-23rd May 1986, vol. 3, pages 759-765, IEEE; J.S. PETROFSKY: "Cyberkinetic assisted walking"<br>* Pages 759-761; figures 3-7 * | 1,2,4, 10,12, 21,22 | A 61 N   1/36<br>A 61 F   5/01 |
| X | US-A-4 569 352  (PETROFSKY)<br>* Column 2, line 23 - column 3, line 64; column 6, lines 41-47; figures 1,2 * | 1,2,4,6 ,8-11 | |
| A | MEDICAL AND BIOLOGICAL ENGINEERING AND COMPUTING, vol. 23, no. 2, March 1985, pages 101-107, IFMBE, London, GB; J. MIZRAHI et al.: "Quantitative weightbearing and gait evaluation of paraplegics using functional electrical stimulation"<br>* Page 102,. left-hand column - page 103, left-hand column; page 103, right-hand column * | 1,5,9- 11,13, 14,19 | |
| A | ENGINEERING IN MEDICINE AND BIOLOGY SOCIETY, PROCEEDINGS OF THE EIGHTH ANNUAL CONFERENCE OF THE IEEE, Fort Worth, Texas, 7th-10th November 1986, vol. 1, pages 675-678, IEEE; D. POPOVIC et al.: "Control methodology for gait restoration"<br>* Page 676, right-hand column - page 677, left-hand column; figures 4,5 * | 1,3,5, 10,14 | TECHNICAL FIELDS SEARCHED (Int. Cl.4)<br><br>A 61 N  ·<br>A 61 F |
| A | US-A-3 881 496  (VREDENBREGT)<br>* Column 2, line 42 - column 3, line 22; figures 1-4 * | 6,8,19 | |

-/-

The present search report has been drawn up for all claims

| Place of search | Date of completion of the search | Examiner |
|---|---|---|
| THE HAGUE | 17-03-1988 | SCHMIERER U.J. |

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | IEEE TRANSACTIONS ON BIOMEDICAL ENGINEERING, vol. BME-33, no. 2, February 1986, pages 256-267, IEEE, New York, US; P.E. CRAGO et al.: "Sensors for use with functional neuromuscular stimulation"<br>* Page 256, right-hand column - page 260, right-hand column; figures 1-4 * | 1,4,6,8,14,19 | |
| A | GB-A-2 139 089 (DREW)<br>* Page 1, lines 16-26; page 1, line 78 - page 2, line 16; figures 1-5 * | 21,23 | |
| A | FR-A-1 552 131 (ECLANCHER)<br>* Column 3, line 48 - column 4, line 36; figures 1,2 * | 15-17, 20 | |
| E | US-A-4 697 808 (LARSON)<br>* Column 3, line 33 - column 4, line 4; column 4, lines 44-52; column 5, lines 11-23; column 6, lines 34-46; column 8, line 41 - column 9, line 2; column 9, lines 16-21; figures 1,2,6,7,10,11,13-15 * | 1-5,7,9,10,13 | |
| E | GB-A-2 186 191 (UNIVERSITY OF STRATHCLYDE)<br>* Page 2, line 102 - page 7, line 45; figures 1-4 * | 1-20 | TECHNICAL FIELDS SEARCHED (Int. Cl.4) |

The present search report has been drawn up for all claims

| Place of search | Date of completion of the search | Examiner |
|---|---|---|
| THE HAGUE | 17-03-1988 | SCHMIERER U.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document